# EUROPEAN PATENT SPECIFICATION

(11) **EP 4 110 197 B1**
(45) Date of publication and mention of the grant of the patent: **12.02.2025**
(21) Application number: 21819598.0
(22) Date of filing: 29.11.2021
(51) Int. Cl.: A61B 17/072, A61B 18/12, A61B 18/14

(54) **POWERED SURGICAL INSTRUMENTS WITH MULTI-PHASE TISSUE TREATMENT**
ANGETRIEBENE CHIRURGISCHE INSTRUMENTE MIT MEHRPHASIGER GEWEBEBEHANDLUNG
INSTRUMENTS CHIRURGICAUX MOTORISÉS AVEC TRAITEMENT DE TISSU À PHASES MULTIPLES

(30) Priority: 02.12.2020 US 202017109589; 02.12.2020 US 202017109669
(43) Date of publication of application: 04.01.2023
(73) Proprietor: Cilag GmbH International, 6300 Zug (CH)
(72) Inventor: SHELTON, IV, Frederick E., Cincinnati, Ohio 45242 (US); FIEBIG, Kevin M., Cincinnati, Ohio 45242 (US); WORTHINGTON, Sarah A., Cincinnati, Ohio 45242 (US); CREAMER, Patrick L., New Orleans, Louisiana 70118 (US); BRADY, John E., Cincinnati, Ohio 45242 (US)
(74) Representative: Carpmaels & Ransford LLP
(86) International application number: PCT/IB2021/061055
(87) International publication number: WO 2022/118164

(56) References cited:
- EP-A1- 2 090 238
- EP-A1- 3 505 095
- US-A- 5 403 312
- US-A1- 2012 209 288
- US-A1- 2015 209 059
- US-A1- 2019 200 998

## Description

### BACKGROUND

The present invention relates to surgical instruments and, in various arrangements, to surgical stapling and cutting instruments and staple cartridges for use therewith that are designed to staple and cut tissue.
EP 3 505 095 A1 describes a surgical instrument which comprises an end effector comprising an ultrasonic blade and a clamp arm. The clamp arm is movable relative to the ultrasonic blade to transition the end effector between an open configuration and a closed configuration to clamp tissue between the ultrasonic blade and the clamp arm. The surgical instrument further comprises a transducer configured to generate an ultrasonic energy output and a waveguide configured to transmit the ultrasonic energy output to the ultrasonic blade. The surgical instrument further comprises a control circuit configured to monitor a parameter of the surgical instrument, wherein crossing an upper predetermined threshold of the parameter causes the control circuit to effect a first electromechanical system, and wherein crossing a lower predetermined threshold of the parameter causes the control circuit to effect a second electromechanical system different than the first electromechanically system.
US 2015/209059 A1 describes methods and devices for controlling motorized surgical devices. In general, the methods and devices can allow a surgical device to grasp and cut tissue. In some embodiments, the device's motor can begin providing power for grasping and/or cutting tissue in response to an output from the device's sensor, the device can adjust power provided by the motor based on whether the device is clamping tissue or is being fired, the device can adjust an amount of power provided by the motor based on an amount of user-applied force to the device's actuator and/or can control drive direction of the motor based on the amount of the force, the device can maintain a force applied to the device, the device can self-shift the motor, and/or the device can adjust an amount of power provided to the device's end effector based on a degree of the end effector's closure.
US 2012/209288 A1 describes systems and methods for displaying an image of a tracked tool on a user interface, wherein at least a portion of the tool is hidden from view in any image on a user interface at any point during a procedure. The tool may include a cutting tool having jaws for clamping a material and a knife for cutting the clamped material, and superimposing an indicator of a knife position on the display during and/or after cutting. The knife position may include a location and/or orientation of the knife, including any or all of a pre-cut, cut-complete, cut-incomplete and an exposed position. The disclosed systems and methods allows a user to view the knife position in applications where the knife may not be visible to a user.
EP 2 090 238 A1 describes a surgical cutting and fastening instrument. The instrument comprises a handle and an end effector connected to the handle. The end effector comprises upper and lower opposing jaw members, wherein the upper jaw member comprises a plurality of colinear, separately actuatable, RF electrodes.
US 2019/2009908 A1 describes a method of adjusting a staple parameter of a surgical stapling instrument. The method includes determining, by a control circuit of the surgical stapling instrument, a first stroke length for a first staple driver of the surgical stapling instrument to drive a first row of staples of a circular stapling head assembly of the surgical stapling instrument; detecting, by the control circuit, a malformed staple in the first row of staples; adjusting, by the control circuit, the staple parameter, based on the detection of the malformed staple; and determining, by the control circuit, a second stroke length for a second staple driver of the surgical stapling instrument to drive a second row of staples of the circular stapling head assembly.
US 5 403 312 A describes an electrosurgical instrument for cauterization and/or welding of tissue of varying impedances, thicknesses and vascularity especially in the performance of endoscopic procedures. The instrument compresses the tissue between one pole of a bipolar energy source located on one interfacing surface, and a second interfacing surface. A second pole is located one of the two interfacing surfaces. In a preferred embodiment, the second pole is located on the same interfacing surface as the first pole and an insulator electrically isolates the two poles. A preferred application of the invention is in a cutting instrument wherein a hemostatic line is formed along a cut line using RF energy.

### SUMMARY

The invention is defined by the independent claims. Further embodiments are defined in the dependent claims.

### BRIEF DESCRIPTION OF THE DRAWINGS

Various features of the embodiments described herein, together with advantages thereof, may be understood in accordance with the following description taken in conjunction with the accompanying drawings as follows:
FIG. 1 is a schematic diagram of a surgical instrument, in accordance with at least one aspect of the present disclosure.
FIG. 2 is a partial perspective view of a jaw of an end effector of the surgical instrument of FIG. 1 and a staple cartridge for assembly therewith.
FIG. 3 is a cross-sectional view of the end effector of the surgical instrument of FIG. 1.
FIG. 4 is cross-sectional view of a tissue that received a surgical treatment from the surgical instrument of FIG. 1.
FIG. 5 is a partial exploded view of an end effector for use with the surgical instrument of FIG. 1, in accordance with at least one aspect of the present disclosure.
FIG. 6 is a partial cross-sectional view of the end effector of FIG. 5 illustrating a channel assembled with a staple cartridge and an radio frequency (RF) overlay, in accordance with at least one aspect of the present disclosure.
FIGS. 7-9 illustrate a process and mechanisms for assembly of the end effector of FIG. 5.
FIG. 10 is a logic flow diagram of a process depicting a control program or a logic configuration for effecting a surgical treatment of a tissue, in accordance with at least one aspect of the present disclosure.
FIG. 11 a graph representing an example implementation of the surgical treatment of the process of FIG. 10 to two tissues with different tissue compressibility.
FIG. 12 is a partial top view a cartridge deck of a cartridge assembled with an end effector of the surgical instrument of FIG. 1.
FIG. 13 is a logic flow diagram of a process depicting a control program or a logic configuration for effecting a surgical treatment of a tissue, in accordance with at least one aspect of the present disclosure.
FIG. 14 is a graph illustrating a sequence for deactivating electrode segments of the end effector of FIG. 1, in accordance with at least one aspect of the present disclosure.
FIG. 15 is a logic flow diagram of a process depicting a control program or a logic configuration for effecting a surgical treatment of a tissue, in accordance with at least one aspect of the present disclosure.

Corresponding reference characters indicate corresponding parts throughout the several views. The exemplifications set out herein illustrate certain embodiments of the invention, in one form, and such exemplifications are not to be construed as limiting the scope of the invention in any manner.

### DETAILED DESCRIPTION

Applicant of the present application also owns the following U.S. Patent Applications that were filed on even date herewith:
- U.S. Patent Application entitled METHOD FOR TISSUE TREATMENT BY SURGICAL INSTRUMENT; Attorney Docket No. END9291USNP1/200802-1M;
- U.S. Patent Application entitled SURGICAL INSTRUMENTS WITH INTERACTIVE FEATURES TO REMEDY INCIDENTAL SLED MOVEMENTS; Attorney Docket No. END9291USNP2/200802-2;
- U.S. Patent Application entitled SURGICAL INSTRUMENTS WITH SLED LOCATION DETECTION AND ADJUSTMENT FEATURES; Attorney Docket No. END9291USNP3/200802-3;
- U.S. Patent Application entitled SURGICAL INSTRUMENT WITH CARTRIDGE RELEASE MECHANISMS; Attorney Docket No. END9291USNP4/200802-4;
- U.S. Patent Application entitled DUAL-SIDED REINFORCED RELOAD FOR SURGICAL INSTRUMENTS; Attorney Docket No. END9291USNP5/200802-5;
- U.S. Patent Application entitled SURGICAL SYSTEMS WITH DETACHABLE SHAFT RELOAD DETECTION; Attorney Docket No. END9291USNP6/200802-6;
- U.S. Patent Application entitled SURGICAL INSTRUMENTS WITH ELECTRICAL CONNECTORS FOR POWER TRANSMISSION ACROSS STERILE BARRIER; Attorney Docket No. END9291USNP7/200802-7;
- U.S. Patent Application entitled DEVICES AND METHODS OF MANAGING ENERGY DISSIPATED WITHIN STERILE BARRIERS OF SURGICAL INSTRUMENT HOUSINGS; Attorney Docket No. END9291USNP8/200802-8;
- U.S. Patent Application entitled POWERED SURGICAL INSTRUMENTS WITH EXTERNAL CONNECTORS; Attorney Docket No. END9291USNP9/200802-9;
- U.S. Patent Application entitled POWERED SURGICAL INSTRUMENTS WITH SMART RELOAD WITH SEPARATELY ATTACHABLE EXTERIORLY MOUNTED WIRING CONNECTIONS; Attorney Docket No. END9291USNP10/200802-10; and
- U.S. Patent Application entitled POWERED SURGICAL INSTRUMENTS WITH COMMUNICATION INTERFACES THROUGH STERILE BARRIER; Attorney Docket No. END9291USNP11/200802-11.

Applicant of the present application owns the following U.S. Patent Applications, filed on December 4, 2018:
• U.S. Patent Application Serial No. 16/209,385, entitled METHOD OF HUB COMMUNICATION, PROCESSING, STORAGE AND DISPLAY;
• U.S. Patent Application Serial No. 16/209,395, entitled METHOD OF HUB COMMUNICATION;
• U.S. Patent Application Serial No. 16/209,403, entitled METHOD OF CLOUD BASED DATA ANALYTICS FOR USE WITH THE HUB;
• U.S. Patent Application Serial No. 16/209,407, entitled METHOD OF ROBOTIC HUB COMMUNICATION, DETECTION, AND CONTROL;
• U.S. Patent Application Serial No. 16/209,416, entitled METHOD OF HUB COMMUNICATION, PROCESSING, DISPLAY, AND CLOUD ANALYTICS;
• U.S. Patent Application Serial No. 16/209,423, entitled METHOD OF COMPRESSING TISSUE WITHIN A STAPLING DEVICE AND SIMULTANEOUSLY DISPLAYING THE LOCATION OF THE TISSUE WITHIN THE JAWS;
• U.S. Patent Application Serial No. 16/209,427, entitled METHOD OF USING REINFORCED FLEXIBLE CIRCUITS WITH MULTIPLE SENSORS TO OPTIMIZE PERFORMANCE OF RADIO FREQUENCY DEVICES;
• U.S. Patent Application Serial No. 16/209,433, entitled METHOD OF SENSING PARTICULATE FROM SMOKE EVACUATED FROM A PATIENT, ADJUSTING THE PUMP SPEED BASED ON THE SENSED INFORMATION, AND COMMUNICATING THE FUNCTIONAL PARAMETERS OF THE SYSTEM TO THE HUB;
• U.S. Patent Application Serial No. 16/209,447, entitled METHOD FOR SMOKE EVACUATION FOR SURGICAL HUB;
• U.S. Patent Application Serial No. 16/209,453, entitled METHOD FOR CONTROLLING SMART ENERGY DEVICES;
• U.S. Patent Application Serial No. 16/209,458, entitled METHOD FOR SMART ENERGY DEVICE INFRASTRUCTURE;
• U.S. Patent Application Serial No. 16/209,465, entitled METHOD FOR ADAPTIVE CONTROL SCHEMES FOR SURGICAL NETWORK CONTROL AND INTERACTION;
• U.S. Patent Application Serial No. 16/209,478, entitled METHOD FOR SITUATIONAL AWARENESS FOR SURGICAL NETWORK OR SURGICAL NETWORK CONNECTED DEVICE CAPABLE OF ADJUSTING FUNCTION BASED ON A SENSED SITUATION OR USAGE;
• U.S. Patent Application Serial No. 16/209,490, entitled METHOD FOR FACILITY DATA COLLECTION AND INTERPRETATION; and
• U.S. Patent Application Serial No. 16/209,491, entitled METHOD FOR CIRCULAR STAPLER CONTROL ALGORITHM ADJUSTMENT BASED ON SITUATIONAL AWARENESS.

Numerous specific details are set forth to provide a thorough understanding of the overall structure, function, manufacture, and use of the embodiments as described in the specification and illustrated in the accompanying drawings. Well-known operations, components, and elements have not been described in detail so as not to obscure the embodiments described in the specification. The reader will understand that the embodiments described and illustrated herein are non-limiting examples, and thus it can be appreciated that the specific structural and functional details disclosed herein may be representative and illustrative. Variations and changes thereto may be made without departing from the scope of the claims.

Various exemplary devices and methods are provided for performing laparoscopic and minimally invasive surgical procedures. However, the reader will readily appreciate that the various methods and devices disclosed herein can be used in numerous surgical procedures and applications including, for example, in connection with open surgical procedures. As the present Detailed Description proceeds, the reader will further appreciate that the various instruments disclosed herein can be inserted into a body in any way, such as through a natural orifice, through an incision or puncture hole formed in tissue, etc. The working portions or end effector portions of the instruments can be inserted directly into a patient's body or can be inserted through an access device that has a working channel through which the end effector and elongate shaft of a surgical instrument can be advanced.

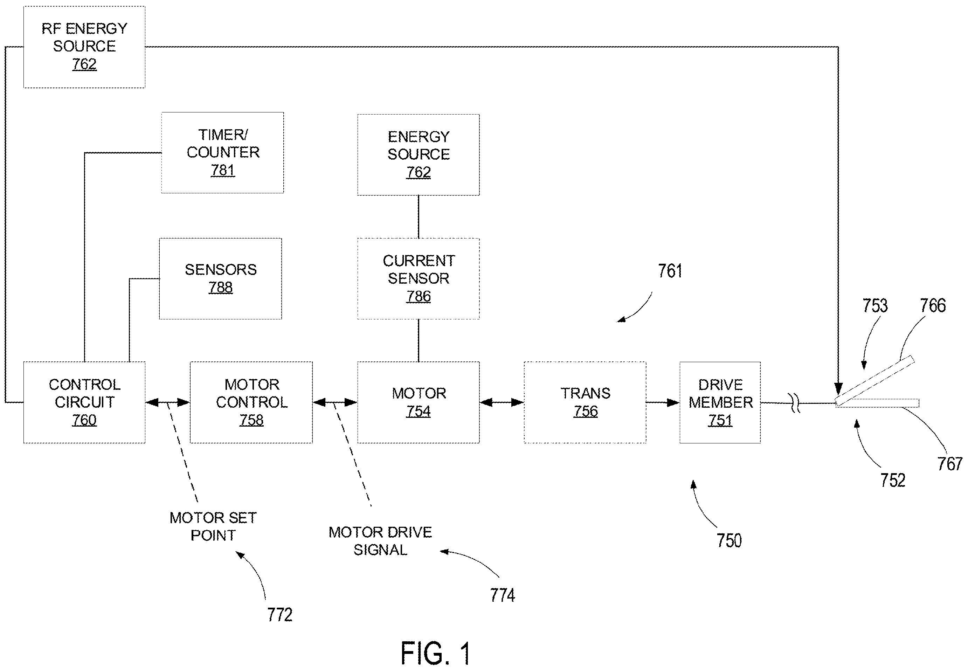

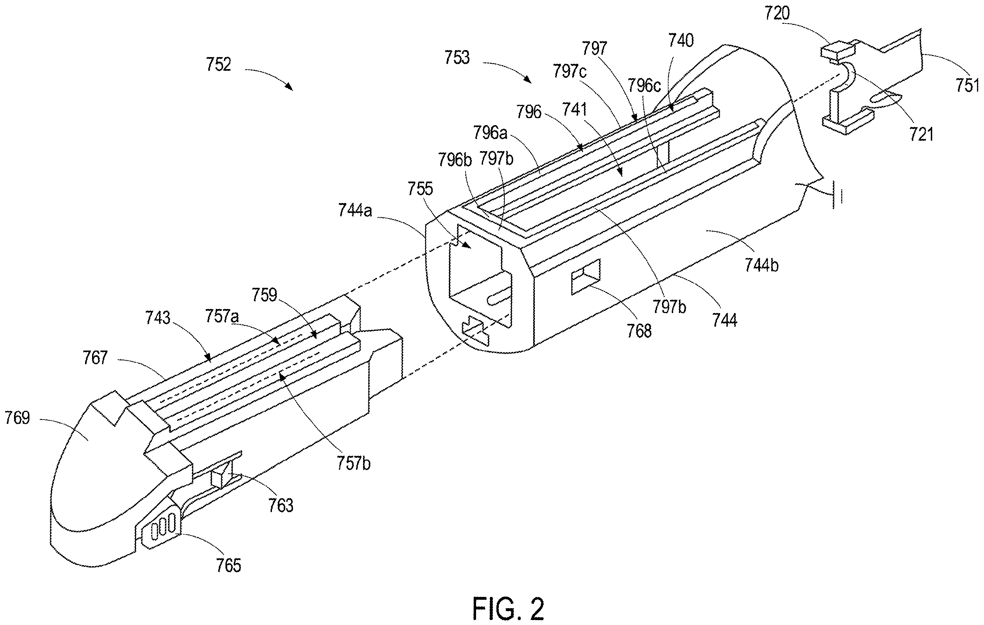

FIG. 1 illustrates a schematic diagram of a surgical instrument 750 configured to deliver a surgical treatment to a tissue to seal and/or cut the tissue. The surgical treatment includes at least two phases. In the first phase, a therapeutic electrical energy is employed to seal the tissue. In at least one example, the therapeutic electrical energy is an RF energy. In the second phase, staples are deployed into the tissue and, optionally, a cutting member 721 (FIG. 2) cuts the tissue.

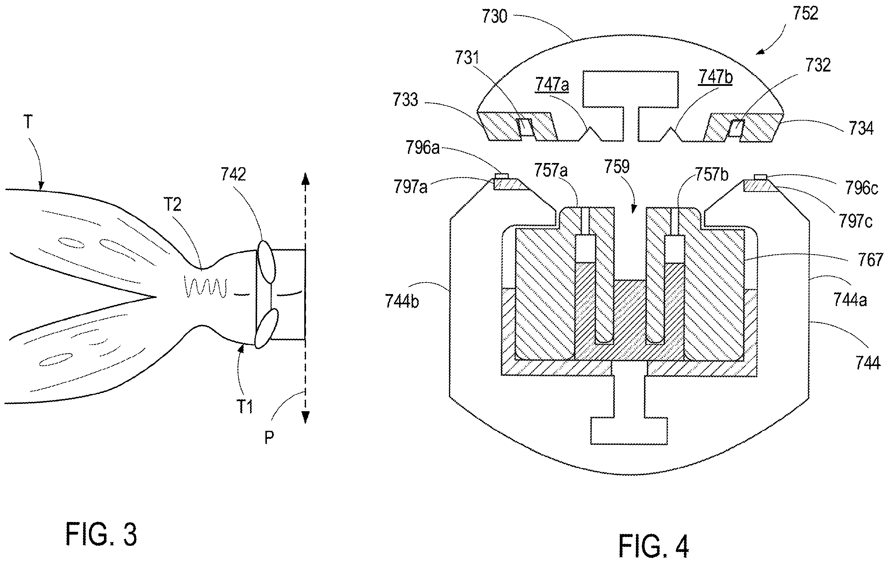

The surgical instrument 750 includes an end effector 752 with jaws 753. At least one of the jaws 753 is movable relative to the other from an open configuration to a closed configuration to grasp tissue therebetween. As illustrated in FIG. 2, the end effector 752 includes at least one electrode 796 configured to deliver the therapeutic electrical energy to the tissue in the first phase of the surgical treatment. The end effector 752 further includes an anvil 766 and a staple cartridge 767 configured to cooperate to form staples 742 (FIG. 3) deployable from the staple cartridge 767 into the tissue in the second phase of the surgical treatment. The staples 742 are formed by anvil pockets 747a, 747b (FIG. 4).

One of the jaws 753 of the end effector 752 includes a channel 744 configured to slidably receive the staple cartridge 767. In the illustrated example, the staple cartridge 767 is inserted into the channel 744 through a distal opening 755. The channel 744 and the staple cartridge 767 include corresponding locking features 763, 768 that cooperate to reversibly lock the staple cartridge 767 and the channel in a locked configuration. In the illustrated example, the locking features 763, 768 are in the form of a ramp and a corresponding groove. In other examples, the locking features 763, 768 can be in the form of protrusions, nubs, bulges, dimples, or any suitable projections, and corresponding valleys, holes, or any suitable depressions. In certain instances, the projections can be in the form of biasing or spring members.

In the illustrated example, the staple cartridge 767 includes two staple cavity rows 757a, 757b on opposite sides of a longitudinal slot 759 configured to accommodate a sliding movement of a cutting member 721. The cutting member 721 is slidably advanced through the longitudinal slot 759 to cut tissue grasped between the jaws 753. In other examples, more or less than two rows of staple cavities can be longitudinally disposed alongside the longitudinal slot 759.

Further to the above, the channel 744 includes a ceiling or cover 740 that includes a longitudinal opening 741 configured to at least partially accommodate the staple cavity rows 757a, 757b when the staple cartridge 767 is assembled with the channel 744. In the illustrated example, the staple cartridge 767 includes a stepped deck 730 that raises the staple cavity rows 757a, 757b. Side walls 744a, 744b of the channel 744 include narrowed portions configured to snuggly receive the stepped deck 730 to ensure a proper alignment of the staple cavity rows 757a, 757b with the longitudinal opening 741 defined in the ceiling or cover 740.

In the illustrated example, the at least one electrode 796 includes electrode segments 796a, 796b, 796c that define a partial perimeter around the longitudinal opening 741. In the assembled configuration, as illustrated in FIG. 4, the raised staple cavity rows 757a, 757b of the stepped deck 730 extend longitudinally in parallel, or substantially in parallel, with the electrode segments 796a, 796b, 796c cooperatively defining a tissue contacting surface. FIG. 3 illustrates a tissue T including a tissue portion T1 fastened by a staple 742 from the staple cavity row 757a, and a tissue portion T2 sealed by RF energy from the electrode segment 796b. The tissue T is cut, in the second phase, along a plane P (perpendicular to the page) by a cutting member 721 driven by an I-beam 720, for example, through the longitudinal slot 759, for example.

In the illustrated example, the electrode segments 796a, 796b, 796c are disposed onto, or are partially embedded, in corresponding insulative segments 797a, 797b, 797c of an insulative layer 797. Furthermore, the anvil 766 includes electrodes 731, 732, which are disposed onto, or are partially embedded, in corresponding insulative segments 733, 734. RF energy may flow from the at least one electrode 796 to the electrodes 731, 732 through tissue grasped between the jaws 753.

To avoid unintentionally forming a short circuit, the electrodes 731, 732 are offset from the electrode segments 797a, 797c, as illustrated in FIG. 4. In other words, the electrodes 731, 732 remain spaced apart from the electrode segments 797a, 797c, respectively, in a closed configuration of the end effector without tissue. In the illustrated example, the channel 744 is grounded, and the at least one electrode 796 is an integral part of the channel 744 with no moving parts. In at least one example, the at least one electrode 796 is hard-wired into channel 744 so electrical connections are not exposed to fluids that may cause a short. In the illustrated example, the electrodes 731, 732 are separated from the anvil 766 by the insulative segments 733, 734, respectively. In other examples, the electrodes 731, 732 are integral with the anvil 766. In such examples, the anvil 766 is a part of the return path of the RF energy.

In the illustrated example, the RF energy is configured to flow from the at least one electrode 796 toward the electrodes 731, 732. In other examples, however, the end effector 752 can be configured to cause the RF energy to flow from the electrodes 731, 732 toward the at least one electrode 796.

When the staple cartridge 767 is assembled with the channel 744, a nose portion 769 of the staple cartridge 767 extend beyond the distal opening 755, while the remainder of the staple cartridge 767 is received within the channel 744. Furthermore, the staple cartridge 767 comprises a cartridge release latch 765 configured to unlock the locking engagement of the locking features 763, 768 to permit removal of the staple cartridge 767 from the channel 744.

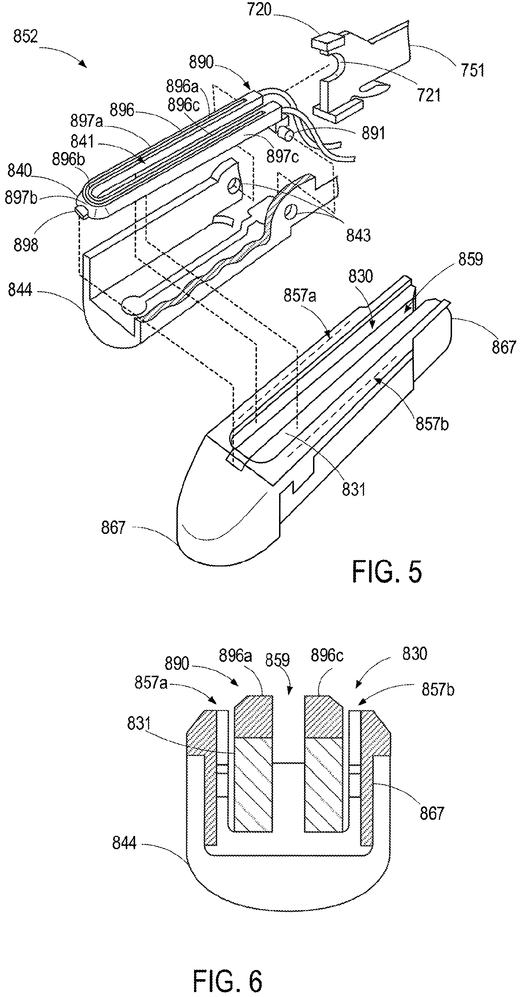

FIGS. 5 and 6 illustrate an end effector 852 similar in many respects to the end effector 752. The end effector 852 can be utilized with the surgical instrument 750 in lieu of the end effector 752. Like the end effector 752, the end effector 852 includes jaws configured to grasp tissue to deliver a surgical treatment to the tissue in first and second treatment phases.

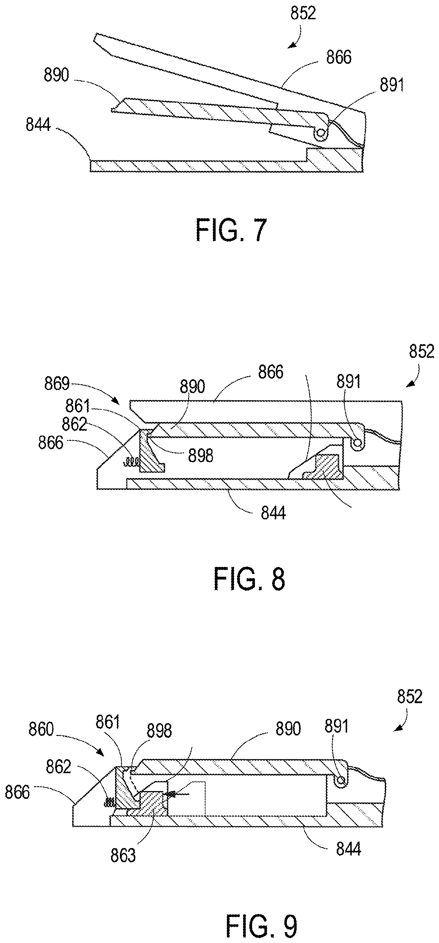

Furthermore, the end effector 852 includes an anvil 866 and a channel 844 configured to releasably retain a staple cartridge 867. An RF overlay 890 is pivotally coupled to the channel 844. FIGS. 7-9 illustrates a process and mechanisms for attaching and detaching the RF overlay 890 to the staple cartridge 867 while the staple cartridge 867 is retained in the channel 844.

In certain examples, the staple cartridge 867, similar to the staple cartridge 767, includes a stepped deck 830 with raised staple cavity rows 857a, 857b and an insulative depressed region 831 configured to releasably retain the RF overlay 890, as best illustrated in FIG. 6. In an assembled configuration, as illustrated in FIG. 6, the staple cavity rows 857a, 857b and at least one electrode 896 of the RF overlay 890 cooperatively define a tissue contacting surface.

Furthermore, the staple cavity rows 857a, 857b and electrode segments 896a, 896b of the at least one electrode 896 extend longitudinally in parallel, or at least substantially in parallel, on opposite sides of a longitudinal slot 859 cooperatively defined by the RF overlay 890 and the staple cartridge 867 while the staple cartridge 867 is retained in the channel 844. In the illustrated example, the drive member 751 terminates in an I-beam 720 that includes a cutting member 721 movable through the longitudinal slot 859 to cut tissue grasped between the jaws of the end effector 852 in a similar manner described in connection with the end effector 752.

In the illustrated example, the RF overlay 890 comprises a U-shape, and includes two body portions 897a, 897c extending longitudinally in parallel, or at least substantially in parallel. The body portions 897a, 897c are separated by a longitudinal opening 841 defined in the RF overlay 890. A distal arcuate portion 897b connects the body portions 897a, 897c. The longitudinal opening 841 facilitates translation of the cutting member 721 relative to the RF overlay 890. The at least one electrode 896 also comprises a U-shape, and is disposed onto, or is at least partially embedded into, the portions 897a, 897b, 897c. In certain instances, the at least one electrode 896 includes 896a, 896b, 896c that can be connected to the RF energy source 762. Other electrode shapes and configurations for the RF overlay 890 are contemplated by the present disclosure.

In the illustrated examples, the overlay 890 includes pivots 891 extending laterally from a proximal portion of the RF overlay 890. The pivots 893 are received in corresponding pivot holes 843 defined in sidewalls of the channel 844. The overlay 890 is rotatable between an unlocked configuration (FIG. 7) and a locked configuration (FIG. 8) about an axis extending through the pivot holes 891. In the illustrated example, the anvil 866 pivots the RF overlay 890 toward the staple cartridge 867. The anvil 866 may cause the staple cartridge 867 to snap into the channel 844, and the RF overlay 890 to snap into the staple cartridge 867.

Furthermore, the staple cartridge 867 includes a latch mechanism 860 including a latch member 861 and a biasing member 862 configured to maintain the latch member 861 at a first position, as illustrated in FIG. 8. In the illustrated example, the RF overlay includes a distal projection 898 configured to be caught by the latch member 861 in the locked configuration.

Further to the above, as illustrated in FIG. 9, a sled 863 can be motivated by the drive member 751 to disengage the latch member 861 from the distal projection 898. In the illustrated example, the sled 863, toward the end of a sled-firing-stroke, pushes the latch member 861 distally, which compresses the biasing member 862, and releases the distal projection 898 from the latch member 861. The spent staple cartridge 867 can then be pulled out of the channel 844, and replaced with an unspent staple cartridge 867. The RF overlay 890 can then be motivated by the anvil 866 into a locking engagement with the unspent staple cartridge 867.

Referring primarily to FIG. 1, a control circuit 760 may be programmed to control one or more functions of the surgical instrument 750 such as, for example, closure of the end effector 752, activation of the at least one electrode, and/or firing the staple cartridge. The control circuit 760, in some examples, may comprise one or more microcontrollers, microprocessors, or other suitable processors for executing instructions that cause the processor or processors to control the one or more functions of the surgical instrument 750. In one aspect, a timer/counter 781 provides an output signal, such as the elapsed time or a digital count, to the control circuit 760. The timer/counter 781 may be configured to measure elapsed time, count external events, or time external events.

The control circuit 760 may generate a motor set point signal 772. The motor set point signal 772 may be provided to a motor controller 758. The motor controller 758 may comprise one or more circuits configured to provide a motor drive signal 774 to the motor 754 to drive the motor 754 as described herein. In some examples, the motor 754 may be a brushed DC electric motor. For example, the velocity of the motor 754 may be proportional to the motor drive signal 774. In some examples, the motor 754 may be a brushless DC electric motor and the motor drive signal 774 may comprise a PWM signal provided to one or more stator windings of the motor 754. Also, in some examples, the motor controller 758 may be omitted, and the control circuit 760 may generate the motor drive signal 774 directly.

The motor 754 may receive power from an energy source 762. The energy source 762 may be or include a battery, a super capacitor, or any other suitable energy source. The motor 754 may be mechanically coupled to the drive member 751 via a transmission 756. The transmission 756 may include one or more gears or other linkage components to couple the motor 754 to a drive member 751.

Further to the above, an RF energy source 762 is coupled to an end effector (e.g., end effectors 752 (FIG. 2), 852 (FIG. 5)), and is applied to an RF electrode of the end effector (e.g., electrodes 796 (FIG. 2), 896 (FIG. 5)) or the RF electrode. In at least one example, the anvil 766 is at least partial made of electrically conductive metal and may be employed as the return path for electrosurgical RF current. The control circuit 760 controls the delivery of the RF energy to the RF electrode 796, or the RF electrode 896.

Additional details are disclosed in U.S. Patent Application Serial No. 15/636,096, entitled SURGICAL SYSTEM COUPLABLE WITH STAPLE CARTRIDGE AND RADIO FREQUENCY CARTRIDGE, AND METHOD OF USING SAME, filed June 28, 2017.

The control circuit 760 may be in communication with one or more sensors 788. The sensors 788 may be positioned on the end effector 752 and adapted to operate with the surgical instrument 750 to measure the various derived parameters such as gap distance versus time, tissue compression versus time, and anvil strain versus time. The sensors 788 may comprise a magnetic sensor, a magnetic field sensor, a strain gauge, a pressure sensor, a force sensor, an inductive sensor such as an eddy current sensor, a resistive sensor, a capacitive sensor, an optical sensor, and/or any other suitable sensor for measuring one or more parameters of the end effector 752.

In one aspect, sensors 788 may be implemented as a limit switch, electromechanical device, solid-state switches, Hall-effect devices, MR devices, GMR devices, magnetometers, among others. In other implementations, the sensors 788 may be solid-state switches that operate under the influence of light, such as optical sensors, IR sensors, ultraviolet sensors, among others. Still, the switches may be solid-state devices such as transistors (e.g., FET, junction FET, MOSFET, bipolar, and the like). In other implementations, the sensors 788 may include electrical conductorless switches, ultrasonic switches, accelerometers, and inertial sensors, among others. The sensors 788 may include one or more sensors.

The control circuit 760 can be configured to simulate the response of the actual system of the instrument in the software of a controller. The drive member 751 can move one or more elements in the end effector 752 at or near a target velocity. The surgical instrument 750 can include a feedback controller, which can be one of any feedback controllers, including, but not limited to a PID, a state feedback, LQR, and/or an adaptive controller, for example. The surgical instrument 750 can include a power source to convert the signal from the feedback controller into a physical input such as case voltage, PWM voltage, frequency modulated voltage, current, torque, and/or force, for example.

As described above in greater detail, various example aspects are directed to a surgical instrument 750 comprising an end effector 752, or an end effector 852, with motor-driven surgical sealing and cutting implements. In various examples, the surgical instrument 750 may comprise a control circuit 760 programmed to control the distal translation of the drive member 751 based on one or more tissue conditions. The control circuit 760 may be programmed to sense tissue conditions, such as thickness, either directly or indirectly, as described herein. The control circuit 760 may be programmed to select a control program based on tissue conditions. A control program may describe the distal motion of the drive member 751. Different control programs may be selected to better treat different tissue conditions. For example, when thicker tissue is present, the control circuit 760 may be programmed to translate the drive member 751 at a lower velocity and/or with lower power. When thinner tissue is present, the control circuit 760 may be programmed to translate the drive member 751 at a higher velocity and/or with higher power.

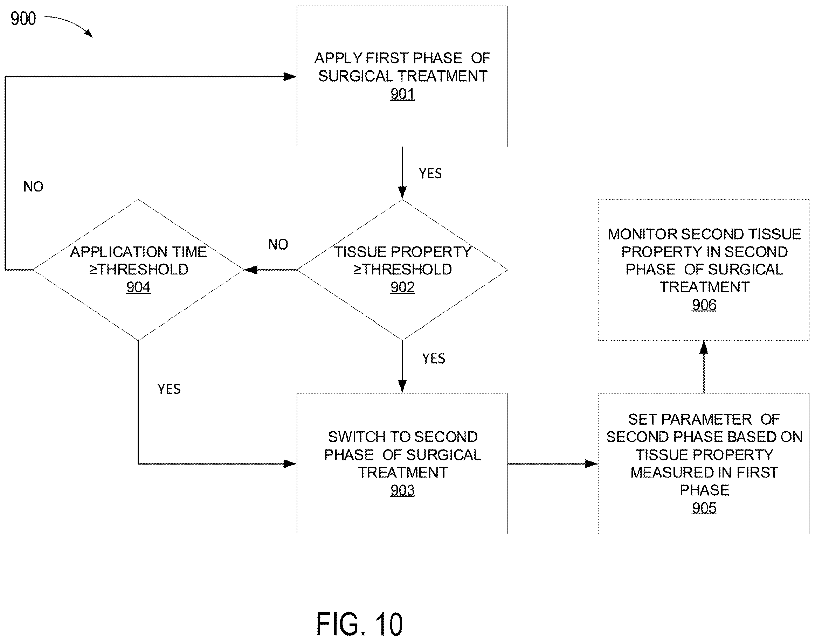

FIG. 10 is a logic flow diagram of a process 900 depicting a control program or a logic configuration for effecting a surgical treatment of a tissue. In certain instances, the process 900 is implemented using the surgical instrument 750, for example. In certain instances the process 900 is implemented, or at least partially implemented, using the control circuit 760, for example. In the illustrated example, the process 900 includes applying 901 a first phase of a surgical treatment to the tissue grasped by the surgical instrument 750. In certain instances, the process 901 includes switching 903 from the first phase of the surgical treatment to the second phase of the surgical treatment based on at least one of a predetermined threshold of the tissue property and a predetermined threshold time of the first phase.

In the illustrated example, if 902 a property of the tissue being treated becomes equal to or greater than a predetermined threshold, the process 901 switches 903 to a second phase of the surgical treatment. The process 904 also switches 903 to the second phase of the surgical treatment if 904 a threshold application time of the first phase is reached prior to the property of the tissue being treated reaching the predetermined threshold. Accordingly, the process 900 may switch from the first phase of the surgical treatment to the second phase of the surgical treatment if at least one of two conditions is met. The first condition is triggered by reaching or exceeding a predetermined threshold of the first tissue property, and the second condition is triggered by reaching or exceeding a predetermined threshold time of the first phase.

In certain instances, the tissue property determined in the first phase is a tissue impedance. Various mechanisms for monitoring tissue impedance are disclosed in U.S. Patent Application Publication No. 20170000553, entitled SURGICAL SYSTEM WITH USER ADAPTABLE TECHNIQUES EMPLOYING MULTIPLE ENERGY MODALITIES BASED ON TISSUE PARAMETERS, filed June 9, 2016. In at least one example, the tissue impedance is determined based on a current passed through the tissue by the RF energy source 762. A current sensor may measure the current passed through the tissue based on a preset voltage value. Alternatively, voltage sensor may measure the voltage between the electrode 796, or alternatively the electrode 896, and a return electrode based on a preset current values. Tissue impedance can be determined based on the current and voltage values.

Further to the above, the first phase and the second phase are different. In at least one example, the first phase comprises an electrical sealing of the tissue, while the second phase comprises a mechanical sealing of the tissue and, optionally, a mechanical cutting of the tissue. In at least one example, the first phase comprises applying a therapeutic RF energy to the tissue, while the second phase comprises stapling the tissue via staples from a staple cartridge. In certain instances, the second phase is applied after completion of the first phase. In other instances, the second phase is set to begin before completion of the first phase. In other instances, the second phase and the first phase are separated by a predetermined wait-time. In certain instances, the wait-time is based on a characteristic of the tissue determined during the first phase.

Further to the above, the process 900 includes setting 905 a parameter of the second phase based on at least one measurement of the tissue property determined in the first phase. In certain examples, the at least one measurement is taken at a beginning of the first phase of the surgical treatment or an end of the first phase of the surgical treatment. In other examples, the at least one measurement comprises multiple measurements of the first tissue property taken during the first phase of the surgical treatment. In one example, the parameter of the second phase is set based on an average of multiple measurements of the first tissue property taken during the first phase of the surgical treatment.

In various aspects, the parameter of the second phase is a drive velocity of the motor controller 758, for example. In certain aspects, the parameter of the second phase is a velocity of the drive member 751, for example. The drive velocity can be an initial drive velocity. In certain instances, the drive velocity is a velocity in a predetermined initial zone of a firing path of the I-beam 720, for example.

The process 900 may further include monitoring 906 a second tissue property, different from the first tissue property, in the second phase of the surgical treatment. In certain instances, the second tissue property is a tissue compression. The sensors 788 may be configured to measure forces exerted on the jaws by a drive member 751 of a drive system 761 of the surgical instrument 750. The forces exerted on the jaws can be representative of the tissue compression experienced by the tissue section grasped by the jaws. The one or more sensors 788 can be positioned at various interaction points along the drive system 761 to detect the closure and/or firing forces applied to the end effector (e.g., end effectors 752, 852) by the drive system 761 (FIG. 1). The one or more sensors 788 may be sampled in real time during the surgical treatment involving a closure/firing operation by the control circuit 760. The control circuit 760 receives real-time sample measurements to provide and analyze time-based information and assess, in real time, closure/firing forces applied to the end effector 752 in the surgical treatment.

In one form, the one or more sensors 788 include a strain gauge sensor that can be used to measure the force applied to the tissue by the end effector, for example. A strain gauge can be coupled to the end effector to measure the force on the tissue being treated by the end effector. In at least one example, the strain gauge sensor is a micro-strain gauge configured to measure one or more parameters of the end effector. In one aspect, the strain gauge sensor can measure the amplitude or magnitude of the strain exerted on a jaw member of an end effector during a surgical treatment, which can be indicative of the tissue compression. The measured strain is converted to a digital signal and provided to the control circuit 760. In certain instances, sensors 788 may comprise a load sensor configured to detect a load generated by the presence of compressed tissue between the jaws of the end effector.

In certain instances, a current sensor 786 can be employed to measure the current drawn by the motor 754. The force required to advance the drive member 751 corresponds to the current drawn by the motor 754. The force is converted to a digital signal and provided to the control circuit 760. The current drawn by the motor 754 can represent tissue compression.

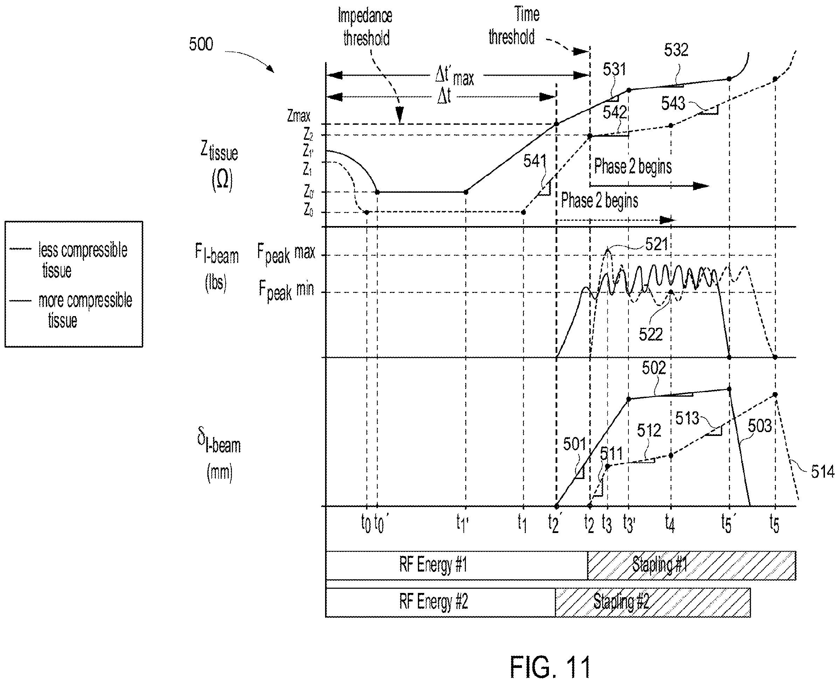

FIG. 11 is a graph 500 representing an example implementation of the surgical treatment of the process 900 to two tissues with different tissue compressibility. A less-compressible tissue is represented by dashed lines, while a more-compressible tissue is represented by solid lines. Graph 500 tracks tissue impedance (Z), I-beam force (F), and I-beam travel distance (δ) against time (t). As RF energy is applied to the tissues, in the first phase of the surgical treatment, the tissue impedance (Z) generally decreases to a minimum value (Z₀ at t₀, Z_{0'} at t_{0'}), which depends, in part, on the compressibility of the tissue. With further application of the RF energy, the minimum tissue impedance is maintained. At t_{1'}, t₁, the tissue impedance begins to rise toward a predetermined maximum threshold of the tissue impedance (Zmax).

The rise of the tissue impedance is faster in the more-compressible tissue than the less-compressible tissue. In the illustrated example, the end of the first phase is determined by reaching, at t_{2'}, a predetermined maximum threshold of the tissue impedance (Zmax) in the case of the more-compressible tissue. However, in the less-compressible tissue, the end of the first phase is determined by reaching, at time t₂, a maximum time threshold (Δt'max) of the first phase. Accordingly, the switch 903 from the first phase to the second phase occurs earlier for the more-compressible tissue, at t_{2'}, than the less-compressible tissue, at t₂.

In the illustrated example, reaching the end of the first phase triggers activation of the motor 754, which begins the second phase of the surgical treatment. The second phase of the surgical treatment involves activating the motor 754 to effect firing a staple cartridge (e.g., staple cartridges 767, 867) by deploying staples from the staple cavity rows into the tissue. The staples are formed against anvil pockets of the anvil (e.g., anvil 766, 866). In the instance of the more-compressible tissue, activation of the motor 754 is triggered by reaching the predetermined maximum threshold of the tissue impedance (Zmax) in the first phase. However, in the instance of the less-compressible tissue, activation of the motor 754 is triggered by reaching the maximum time threshold (Δt'max) of the first phase at time t₂.

Further to the above, different initial I-beam or motor drive velocities V0', V0 (slopes of lines 501, 511) are selected for the second phase based on the tissue impedance readings determined at the ends (t_{2'}, t₂) of the first phase, as determined by reaching the predetermined maximum threshold of the tissue impedance (Zmax), or by reaching the maximum time threshold (Δt'max). In other examples, the initial I-beam or motor drive velocities V0', V0 (slopes of lines 501, 511) of the second phase can be determined based on tissue impedance readings at the beginnings (Z1', Z1) of the first phase. In yet other examples, the initial I-beam or motor drive velocities V0', V0 (slopes of lines 501, 511) of the second phase can be determined based on multiple tissue impedance readings at various points of the first phase. For example, and average of multiple tissue impedance readings at various points of the first phase can be used to determine an initial I-beam or motor drive velocity of the second phase.

In various instances, the control circuit 760 includes a microcontroller with a storage medium and a processor. The storage medium may be in the form of a memory unit storing a database, an equation, or a lookup table that can be utilized by the processor to determine an initial I-beam or motor drive velocity for the second phase based on tissue impedance readings of the first phase. In certain instances, the initial I-beam or motor drive velocity is an initial steady state velocity after an initial ramping segment to reach the initial steady state velocity. In certain instances, the initial I-beam or motor drive velocity is a target initial velocity set by the processor based on the tissue impedance readings of the first phase.

Referring still to FIG. 11, as described above in greater detail, the process 900 may include monitoring 906 tissue compression during the second phase, and making adjustments to the I-beam or motor drive velocity based on the detected tissue compression. In the illustrated example, the control circuit 760 is configured to maintain the I-beam force within a predetermined force threshold range (Fmin-Fmax). The I-beam force in the instance of the less-compressible tissue reaches 521 the predetermined maximum I-beam force (Fmax) at t₃, which triggers the control circuit 760 to adjust the drive velocity of the I-beam or motor. In the illustrated example, the control circuit 760 adjusts the drive velocity from the initial drive velocity V₀ (slope of the line 511) to a drive velocity V₁ (slope of the line 512) less than the initial drive velocity V₀. The reduction in drive velocity at t₃ causes the I-beam force to decrease to a level below the predetermined maximum I-beam force (Fmax).

Conversely, at t₄, the I-beam force in the instance of the less-compressible tissue, reaches 522 the predetermined minimum I-beam force (Fmin), which triggers the control circuit 760 to adjust the drive velocity of the I-beam or motor. In the illustrated example, the control circuit 760 adjusts the drive velocity from the drive velocity V₁ (slope of the line 512) to a drive velocity V₂ (slope of the line 513) greater than the drive velocity V₁. The increase in drive velocity at t₄ causes the I-beam force to increase to a level above the predetermined minimum I-beam force (Fmin), and remain within the predetermined force threshold range (Fmin-Fmax).

In addition to making adjustments to the drive velocity based on the I-beam force, the control circuit 760 may also make adjustments to the drive velocity based on the tissue impedance readings determined within the second phase. In certain instances, the tissue impedance is monitored in the second phase by driving a non-therapeutic, or therapeutic, current through the tissue, and measuring the tissue impedance based on the non-therapeutic, or therapeutic, current. In certain instances, the adjustments to the drive velocity based on the tissue impedance readings within the second phase are performed while the I-beam force is maintained within the predetermined force threshold range (Fmin-Fmax). Accordingly, in such instances, the control circuit 760 is configured to make first adjustments to the drive velocity based on the tissue compression, and second adjustments to the drive velocity based on the tissue impedance.

In certain instances, the adjustments to the drive velocity during the second phase can be based on the rate of change of the tissue impedance. As the I-beam is advanced distally, the tissue compression causes changes in tissue impedance over time. In the illustrated example, the control circuit 760 determines the rate of change of tissue impedance (ΔZ₁/Δt₁), e.g., slope of the line 531, by monitoring changes in tissue impedance over time, e.g., time period t_{2'}-t_{3'}.

If the control circuit 760 determines that the rate of change of the tissue impedance is beyond a predetermined threshold range, the control circuit 760 may adjust the drive velocity to return the rate of change of the tissue impedance to a value within the predetermined threshold range. For example, the drive velocity may be adjusted from the initial drive velocity V0', slope of the line 501, to a drive velocity V1', slope of the line 502, which causes the rate of change of the tissue impedance to be adjusted from (ΔZ₁/Δt₁), slope of the line 531, to (ΔZ₂/Δt₂), slope of the line 532.

In certain instances, the rate of change of the tissue impedance in the second phase is utilized as a feedback indicator for drive velocity adjustments. The adjustments in the drive velocity can yield changes in the rate of change of the tissue impedance. In the illustrated examples, slopes of the lines 541, 542, 543 correspond to the slopes of the lines 511, 512, 513, for example. Accordingly, a control circuit 760 can be configured to confirm changes made to the drive velocity settings by monitoring the rate of change of the tissue impedance, for example.

Further to the above, still referring to FIG. 11, the second phase of the surgical treatment involves firing a staple cartridge (e.g., staple cartridges 767, 867) by deploying staples from the staple cavity rows into the tissue. The staples are formed against anvil pockets of the anvil (e.g., anvil 766, 866). As the staples are gradually deployed and formed, the I-beam force fluctuates within the predetermined force threshold range (Fmin-Fmax). As described above, the control circuit 760 is configured to maintain the I-beam force within the predetermined force threshold range (Fmin-Fmax) by making adjustments to the drive velocity. Toward the end of the second phase, after the staple deployment and forming is completed, the I-beam force rapidly decreases to a minimum value, which coincides with a rapid increase in the tissue impedance curve (e.g., at t₅, t_{5'}), which can be detected by the control circuit 760 based on tissue impedance readings. In response, the control circuit 760 further adjusts the drive velocity (e.g., slopes of lines 503, 514) to terminate the second phase.

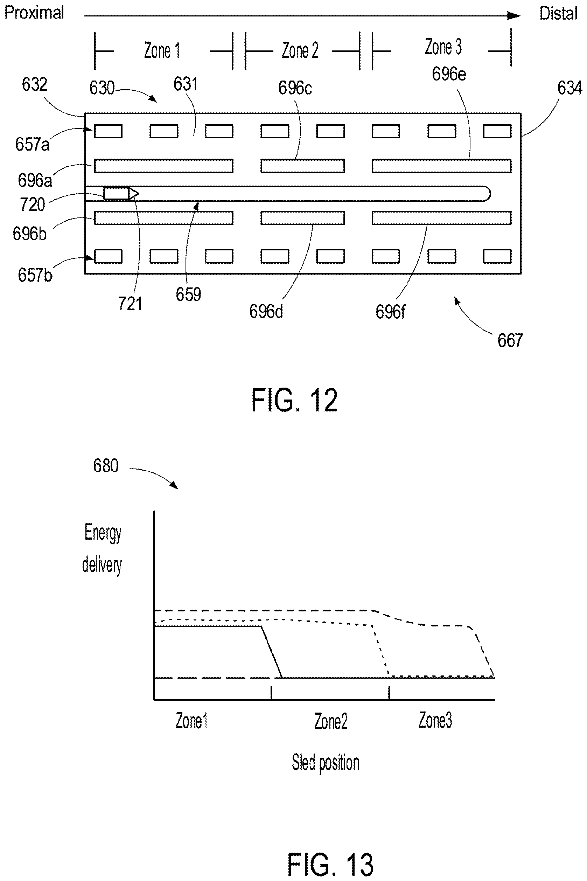

Referring now to FIG. 12, a top view of a cartridge deck 630 is represented. The cartridge deck 630 is similar in many respects to other cartridge decks disclosed elsewhere herein such as, for example, the cartridge decks 730, 830. For example, the cartridge deck 630 includes two staple cavity rows 657a, 657b on opposite sides of a longitudinal slot 659. Furthermore, the cartridge deck 630 also includes electrode segments 696a, 696c, 696e and electrode segments 696b, 696d, 696f on opposite sides of the longitudinal slot 659.

In the illustrated example, the staple cavity rows 657a, 657b are closer to the longitudinal slot 659 than the electrode segments 696a-696f. In other arrangements, however, the staple cavity rows 657a, 657b can be further away from the longitudinal slot 659 than the electrode segments 696a-696f. In various instances, a cartridge deck 630 may include more, or less, than two staple cavity rows and/or more, or less, than six electrode segments.

In certain instances, the cartridge deck 630 can be implemented using an end effector similar in many respects to the end effector 752 (FIG. 2). In such instances, the electrode segments 696a-696f can be integrated with a channel 744 (FIG. 2), for example. The cartridge deck 630 can be formed by insertion of a staple cartridge including the staple cavity rows 657a, 657b into a distal end of the channel 744.

In other instances, the cartridge deck 630 can be implemented using an end effector similar in many respects to the end effector 852 (FIG. 5). In such instances, the electrode segments 696a-696f can be integrated into an RF overlay, similar in many respects to the RF overlay 890. Further, the cartridge deck 630 can be formed by insertion of a staple cartridge including the staple cavity rows 657a, 657b into a channel 844, and pivoting the RF overlay that includes the electrode segments 696a-696f toward the channel 844, and into a locking engagement with the staple cartridge, as detailed by the assembly process described in connection with FIGS. 7-9.

Further to the above, the cartridge deck 630 may form a tissue contacting surface 631 for grasping tissue in cooperation with an anvil 766, for example, and in response to drive motions generated by the motor 754 of the surgical instrument 750, for example. Furthermore, the electrode segments 696a-696f can be electrically coupled to the RF energy source 762, which can selectively transmit RF energy to the tissue grasped between the tissue contacting surface 631 of the cartridge deck 630 and the anvil 766. The control circuit 760 may cause the RF energy source 762 to selectively energize and de-energize, or activate and deactivate, the electrode segments 696a-696f in a predetermined sequence to deliver a therapeutic RF energy to the grasped tissue.

In the illustrated example, the electrode segments 696a-696f are arranged in two rows on opposite sides of the longitudinal slot 659. The electrode segments in each row are separately residing in consecutive treatment zones: a proximal zone (Zone 1), an intermediate zone (Zone 2), and a distal zone (Zone 3), for example. In other examples, more or less than three consecutive treatment zones are contemplated such as, for example, two, four, five, and/or size treatment zones.

In the illustrated example, the electrode segments 696a-696f are arranged are arranged in pairs in each of the consecutive treatment zones. The electrode segments of a pair (e.g., electrode segments 695a, 696b) are positioned on opposite sides of the longitudinal slot 659. In other examples, electrode segments in the consecutive treatment zones can be arranged on one side of the longitudinal slot 659. In other examples, electrode segments in the consecutive treatment zones could alternate where a first electrode segment resides in a first treatment zone on one side of the longitudinal slot 659, while a second electrode segment resides in a second treatment zone, distal, or proximal, to the first treatment zone, on the other side of the longitudinal slot 659.

In the illustrated example, the electrode segments 696a-696f are different in size. Specifically, the electrode segments 696c, 696d of the intermediate zone are smaller in size than the electrode segments 696a, 696b, 696e, 696f in the proximal and distal zones. In other examples, electrode segments with different, or the same, sizes are contemplated. In one example, electrode segments arranged in a row may comprise sizes increasing gradually in a proximal direction or a distal direction.

In the illustrated example, the electrode segments of different treatment zones are spaced apart and can be separately activated, or deactivated, in a predetermined sequence. In at least one example, each electrode segment, or pair of electrode segments, in a treatment zone is separately coupled to the RF energy source thereby allowing the RF energy source 762 to selectively energize and de-energize, or activate and deactivate, the electrode segments 696a-696f in a predetermined sequence to selectively deliver a therapeutic RF energy to the grasped tissue in a predetermined zone-treatment order, as discussed in greater detail below.

In addition to the RF energy, staples from the staple cavity rows 657a, 657b are deployed into the tissue. The staples are formed against anvil pockets of the anvil (e.g., anvil 766, 866). The staples are sequentially deployed by a sled driven by the I-beam 720 and advanced from a proximal end 632 toward a distal end 634 of the cartridge deck 630. The sled advancement by the I-beam 720 is motivated by drive motions generated by the motor 754 and transmitted to the I-beam 720 by the drive member 751, for example.

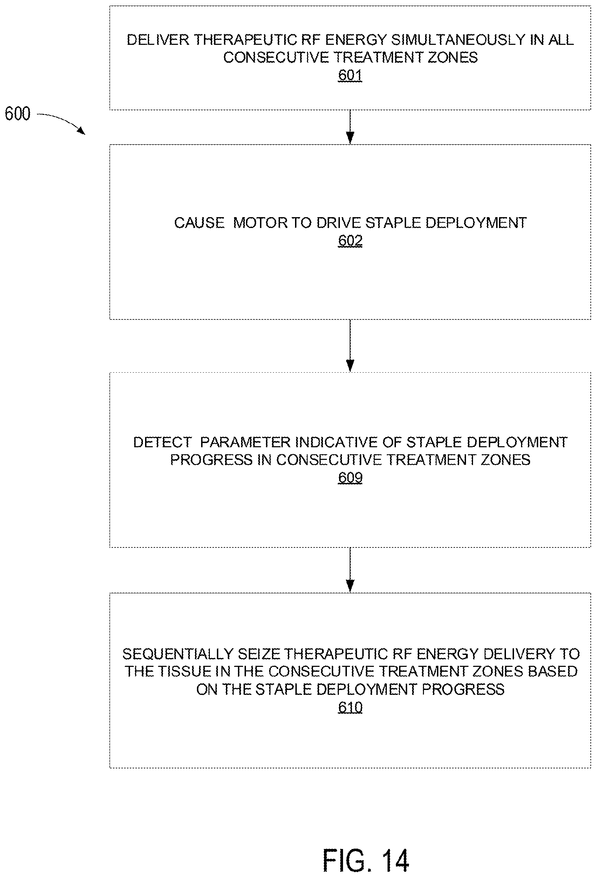

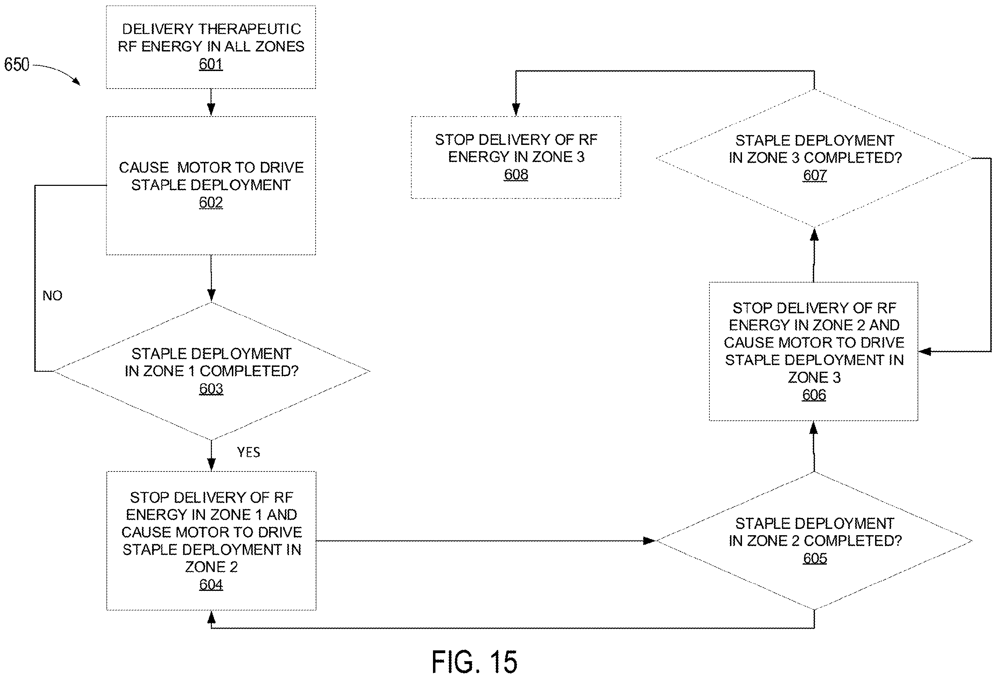

FIGS. 14 and 15 are logic flow diagrams of processes 600, 650 depicting control programs or logic configurations for effecting surgical treatments of tissue. In one form, the processes 600, 650 are implemented by the surgical instrument 750 while equipped with an end effector including the cartridge deck 630 (FIG. 12), for example. The tissue is grasped between the tissue contacting surface 631 of the cartridge deck 630 of a staple cartridge 667 and an anvil 766 (FIG. 1), for example.

The processes 600, 650 include simultaneously delivering 601 a therapeutic energy to the tissue in all the consecutive treatment zones. The processes 600, 650 further include causing 602 the motor 754 to drive staple deployment from the staple cartridge 667 sequentially in the consecutive treatment zones residing between the proximal end 632 and the distal end 634 of the cartridge deck 630.

The process 600 includes detecting 609 a parameter indicative of progress of the staple deployment from the staple cartridge in the consecutive treatment zones, and sequentially deactivating electrode segments 696a-696f to sequentially seize 610 the delivery of the therapeutic energy to the tissue in the consecutive treatment zones based on the progress of the staple deployment from the staple cartridge.

In at least one example, as illustrated in FIG. 15, and as illustrated in a graph 680 of FIG. 13, the process 650 continues to deliver the therapeutic RF energy to Zone1, Zone2, and Zone 3 until certain conditions are met. If 603 it is detected that the staple deployment in Zone 1 is completed, the process 650 stops 604 delivery of the therapeutic RF energy to Zone 1, while continuing to deliver the therapeutic RF energy to Zone 2 and Zone 3. Then, if 605 it is detected that the staple deployment in Zone 2 is completed, the process 650 stops 606 delivery of the therapeutic RF energy to Zone 2, while continuing to deliver the therapeutic RF energy to Zone 3. Finally, if 607 it is detected that the staple deployment in Zone 3 is completed, the process 650 stops 608 delivery of the therapeutic RF energy to Zone 3.

In certain instances, the parameter indicative of the progress of the staple deployment is a distance-based parameter or a position-based parameter. In such instances, the control circuit 760 is configured to implement a predetermined deactivation sequence of the electrode segments 696a-696f based on the progress of the staple deployment, as detected based on distance and/or position readings received from one or more sensors 788.

The distance can be a distance travelled by the drive member 751 or the I-beam 720 to advance a sled, for example, through the consecutive treatment zones. Likewise, the position can be a position of the I-beam 720, or a sled driven by the I-beam 720, with respect to the consecutive treatment zones. In certain instances, detecting that the I-beam 720 has transitioned from a proximal zone to a distal zone triggers the control circuit 760 to seize the delivery of the therapeutic RF energy to the proximal zone.

In various aspects, the one or more sensors 788 may include a position sensor configured to sense a position of the drive member 751 and/or I-beam 720, for example. The position sensor may be or include any type of sensor that is capable of generating position data that indicate a position of the drive member 751 and/or I-beam 720. In some examples, the position sensor may include an encoder configured to provide a series of pulses to the control circuit 760 as the drive member 751 and/or I-beam 720 translates distally and proximally. The control circuit 760 may track the pulses to determine the position of the drive member 751 and/or I-beam 720. Other suitable position sensors may be used, including, for example, a proximity sensor. Other types of position sensors may provide other signals indicating motion of the drive member 751 and/or I-beam 720.

In certain instances, where the motor 754 is a stepper motor, the control circuit 760 may track the position of the drive member 751 by aggregating the number and direction of steps that the motor 754 has been instructed to execute. Accordingly, in such instances, the parameter indicative of the progress of the staple deployment can be based on the number and direction of steps that the motor 754 has been instructed to execute.

The position sensor may be located in the end effector 752 or at any other portion of the instrument. Further, a detailed description of an absolute positioning system, for use with the surgical instrument 750, is described in U.S. Patent Application Publication No. 2017/0296213, entitled SYSTEMS AND METHODS FOR CONTROLLING A SURGICAL STAPLING AND CUTTING INSTRUMENT, which published on October 19, 2017.

In certain instances, the parameter indicative of the progress of the staple deployment is a time-based parameter. The control circuit 760 may employ the timer/counter 781 to assess the staple deployment progress, for example. The control circuit 760 may start the timer/counter 781 and activate the motor 754 (FIG. 1) simultaneously. The control circuit 760 utilizes the time spent after activation of the motor 754, as detected by the timer/counter 781, to assess the staple deployment progress based on a technique, an equation, a formula, a database, and/or a lookup table stored in a memory unit, for example.

In certain instances, the parameter indicative of the progress of the staple deployment is a tissue impedance-based parameter or a force-based parameter. In certain instances, the parameter indicative of the progress of the staple deployment is based on tissue thickness, for example.

Measurements of the tissue compression, the tissue impedance, the tissue thickness, and/or the force required to close the end effector on the tissue, as measured by the sensors 788, can be used by a microcontroller of the control circuit 760 to assess the staple deployment progress, for example. In one instance, the microcontroller may include a memory that stores a technique, an equation, a formula, a database, and/or a lookup table, which can be employed by the microcontroller to assess the staple deployment progress based on readings from the sensors 788.

The surgical instrument systems described herein are motivated by an electric motor; however, the surgical instrument systems described herein can be motivated in any suitable manner. In certain instances, the motors disclosed herein may comprise a portion or portions of a robotically controlled system. U.S. Patent Application Serial No. 13/118,241, entitled SURGICAL STAPLING INSTRUMENTS WITH ROTATABLE STAPLE DEPLOYMENT ARRANGEMENTS, now U.S. Patent No. 9,072,535, for example, discloses several examples of a robotic surgical instrument system in greater detail. The disclosures of International Patent Publication No. WO 2017/083125, entitled STAPLER WITH COMPOSITE CARDAN AND SCREW DRIVE, published May 18, 2017, International Patent Publication No. WO 2017/083126, entitled STAPLE PUSHER WITH LOST MOTION BETWEEN RAMPS, published May 18, 2017, International Patent Publication No. WO 2015/153642, entitled SURGICAL INSTRUMENT WITH SHIFTABLE TRANSMISSION, published October 8, 2015, U.S. Patent Application Publication No. 2017/0265954, filed March 17, 2017, entitled STAPLER WITH CABLE-DRIVEN ADVANCEABLE CLAMPING ELEMENT AND DUAL DISTAL PULLEYS, U.S. Patent Application Publication No. 2017/0265865, filed February 15, 2017, entitled STAPLER WITH CABLE-DRIVEN ADVANCEABLE CLAMPING ELEMENT AND DISTAL PULLEY, and U.S. Patent Application Publication No. 2017/0290586, entitled STAPLING CARTRIDGE, filed on March 29, 2017.

The surgical instrument systems described herein have been described in connection with the deployment and deformation of staples; however, the embodiments described herein are not so limited. Various embodiments are envisioned which deploy fasteners other than staples, such as clamps or tacks, for example. Moreover, various embodiments are envisioned which utilize any suitable means for sealing tissue. For instance, an end effector in accordance with various embodiments can comprise electrodes configured to heat and seal the tissue. Also, for instance, an end effector in accordance with certain embodiments can apply vibrational energy to seal the tissue.

The foregoing detailed description has set forth various forms of the devices and/or processes via the use of block diagrams, flowcharts, and/or examples. Insofar as such block diagrams, flowcharts, and/or examples contain one or more functions and/or operations, it will be understood by those within the art that each function and/or operation within such block diagrams, flowcharts, and/or examples can be implemented, individually and/or collectively, by a wide range of hardware, software, firmware, or virtually any combination thereof. Those skilled in the art will recognize that some aspects of the forms disclosed herein, in whole or in part, can be equivalently implemented in integrated circuits, as one or more computer programs running on one or more computers (e.g., as one or more programs running on one or more computer systems), as one or more programs running on one or more processors (e.g., as one or more programs running on one or more microprocessors), as firmware, or as virtually any combination thereof, and that designing the circuitry and/or writing the code for the software and or firmware would be well within the skill of one of skill in the art in light of this disclosure. In addition, those skilled in the art will appreciate that the mechanisms of the subject matter described herein are capable of being distributed as one or more program products in a variety of forms, and that an illustrative form of the subject matter described herein applies regardless of the particular type of signal bearing medium used to actually carry out the distribution.

Instructions used to program logic to perform various disclosed aspects can be stored within a memory in the system, such as dynamic random access memory (DRAM), cache, flash memory, or other storage. Furthermore, the instructions can be distributed via a network or by way of other computer readable media. Thus a machine-readable medium may include any mechanism for storing or transmitting information in a form readable by a machine (e.g., a computer), but is not limited to, floppy diskettes, optical disks, compact disc, read-only memory (CD-ROMs), and magneto-optical disks, read-only memory (ROMs), random access memory (RAM), erasable programmable read-only memory (EPROM), electrically erasable programmable read-only memory (EEPROM), magnetic or optical cards, flash memory, or a tangible, machine-readable storage used in the transmission of information over the Internet via electrical, optical, acoustical or other forms of propagated signals (e.g., carrier waves, infrared signals, digital signals, etc.). Accordingly, the non-transitory computer-readable medium includes any type of tangible machine-readable medium suitable for storing or transmitting electronic instructions or information in a form readable by a machine (e.g., a computer).

As used in any aspect herein, the term "control circuit" may refer to, for example, hardwired circuitry, programmable circuitry (e.g., a computer processor including one or more individual instruction processing cores, processing unit, processor, microcontroller, microcontroller unit, controller, digital signal processor (DSP), programmable logic device (PLD), programmable logic array (PLA), or field programmable gate array (FPGA)), state machine circuitry, firmware that stores instructions executed by programmable circuitry, and any combination thereof. The control circuit may, collectively or individually, be embodied as circuitry that forms part of a larger system, for example, an integrated circuit (IC), an application-specific integrated circuit (ASIC), a system on-chip (SoC), desktop computers, laptop computers, tablet computers, servers, smart phones, etc. Accordingly, as used herein "control circuit" includes, but is not limited to, electrical circuitry having at least one discrete electrical circuit, electrical circuitry having at least one integrated circuit, electrical circuitry having at least one application specific integrated circuit, electrical circuitry forming a general purpose computing device configured by a computer program (e.g., a general purpose computer configured by a computer program which at least partially carries out processes and/or devices described herein, or a microprocessor configured by a computer program which at least partially carries out processes and/or devices described herein), electrical circuitry forming a memory device (e.g., forms of random access memory), and/or electrical circuitry forming a communications device (e.g., a modem, communications switch, or optical-electrical equipment). Those having skill in the art will recognize that the subject matter described herein may be implemented in an analog or digital fashion or some combination thereof.

As used in any aspect herein, the term "logic" may refer to an app, software, firmware and/or circuitry configured to perform any of the aforementioned operations. Software may be embodied as a software package, code, instructions, instruction sets and/or data recorded on non-transitory computer readable storage medium. Firmware may be embodied as code, instructions or instruction sets and/or data that are hard-coded (e.g., nonvolatile) in memory devices.

As used in any aspect herein, the terms "component," "system," "module" and the like can refer to a computer-related entity, either hardware, a combination of hardware and software, software, or software in execution.

As used in any aspect herein, an "algorithm" refers to a self-consistent sequence of steps leading to a desired result, where a "step" refers to a manipulation of physical quantities and/or logic states which may, though need not necessarily, take the form of electrical or magnetic signals capable of being stored, transferred, combined, compared, and otherwise manipulated. It is common usage to refer to these signals as bits, values, elements, symbols, characters, terms, numbers, or the like. These and similar terms may be associated with the appropriate physical quantities and are merely convenient labels applied to these quantities and/or states.

A network may include a packet switched network. The communication devices may be capable of communicating with each other using a selected packet switched network communications protocol. One example communications protocol may include an Ethernet communications protocol which may be capable permitting communication using a Transmission Control Protocol/Internet Protocol (TCP/IP). The Ethernet protocol may comply or be compatible with the Ethernet standard published by the Institute of Electrical and Electronics Engineers (IEEE) titled "IEEE 802.3 Standard", published in December, 2008 and/or later versions of this standard. Alternatively or additionally, the communication devices may be capable of communicating with each other using an X.25 communications protocol. The X.25 communications protocol may comply or be compatible with a standard promulgated by the International Telecommunication Union-Telecommunication Standardization Sector (ITU-T). Alternatively or additionally, the communication devices may be capable of communicating with each other using a frame relay communications protocol. The frame relay communications protocol may comply or be compatible with a standard promulgated by Consultative Committee for International Telegraph and Telephone (CCITT) and/or the American National Standards Institute (ANSI). Alternatively or additionally, the transceivers may be capable of communicating with each other using an Asynchronous Transfer Mode (ATM) communications protocol. The ATM communications protocol may comply or be compatible with an ATM standard published by the ATM Forum titled "ATM-MPLS Network Interworking 2.0" published August 2001, and/or later versions of this standard. Of course, different and/or after-developed connection-oriented network communication protocols are equally contemplated herein.

Unless specifically stated otherwise as apparent from the foregoing disclosure, it is appreciated that, throughout the foregoing disclosure, discussions using terms such as "processing," "computing," "calculating," "determining," "displaying," or the like, refer to the action and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

One or more components may be referred to herein as "configured to," "configurable to," "operable/operative to," "adapted/adaptable," "able to," "conformable/conformed to," etc. Those skilled in the art will recognize that "configured to" can generally encompass active-state components and/or inactive-state components and/or standby-state components, unless context requires otherwise.

The terms "proximal" and "distal" are used herein with reference to a clinician manipulating the handle portion of the surgical instrument. The term "proximal" refers to the portion closest to the clinician and the term "distal" refers to the portion located away from the clinician. It will be further appreciated that, for convenience and clarity, spatial terms such as "vertical", "horizontal", "up", and "down" may be used herein with respect to the drawings. However, surgical instruments are used in many orientations and positions, and these terms are not intended to be limiting and/or absolute.

Those skilled in the art will recognize that, in general, terms used herein, and especially in the appended claims (e.g., bodies of the appended claims) are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to claims containing only one such recitation, even when the same claim includes the introductory phrases "one or more" or "at least one" and indefinite articles such as "a" or "an" (e.g., "a" and/or "an" should typically be interpreted to mean "at least one" or "one or more"); the same holds true for the use of definite articles used to introduce claim recitations.

In addition, even if a specific number of an introduced claim recitation is explicitly recited, those skilled in the art will recognize that such recitation should typically be interpreted to mean at least the recited number (e.g., the bare recitation of "two recitations," without other modifiers, typically means at least two recitations, or two or more recitations). Furthermore, in those instances where a convention analogous to "at least one of A, B, and C, etc." is used, in general such a construction is intended in the sense one having skill in the art would understand the convention (e.g., "a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (e.g., "a system having at least one of A, B, or C" would include but not be limited to systems that have A alone, B alone, C alone, A and B together, A and C together, B and C together, and/or A, B, and C together, etc.). It will be further understood by those within the art that typically a disjunctive word and/or phrase presenting two or more alternative terms, whether in the description, claims, or drawings, should be understood to contemplate the possibilities of including one of the terms, either of the terms, or both terms unless context dictates otherwise. For example, the phrase "A or B" will be typically understood to include the possibilities of "A" or "B" or "A and B."

With respect to the appended claims, those skilled in the art will appreciate that recited operations therein may generally be performed in any order. Also, although various operational flow diagrams are presented in a sequence(s), it should be understood that the various operations may be performed in other orders than those which are illustrated, or may be performed concurrently. Examples of such alternate orderings may include overlapping, interleaved, interrupted, reordered, incremental, preparatory, supplemental, simultaneous, reverse, or other variant orderings, unless context dictates otherwise. Furthermore, terms like "responsive to," "related to," or other past-tense adjectives are generally not intended to exclude such variants, unless context dictates otherwise.

It is worthy to note that any reference to "one aspect," "an aspect," "an exemplification," "one exemplification," and the like means that a particular feature, structure, or characteristic described in connection with the aspect is included in at least one aspect. Thus, appearances of the phrases "in one aspect," "in an aspect," "in an exemplification," and "in one exemplification" in various places throughout the specification are not necessarily all referring to the same aspect. Furthermore, the particular features, structures or characteristics may be combined in any suitable manner in one or more aspects.

In this specification, unless otherwise indicated, terms "about" or "approximately" as used in the present disclosure, unless otherwise specified, means an acceptable error for a particular value as determined by one of ordinary skill in the art, which depends in part on how the value is measured or determined. In certain embodiments, the term "about" or "approximately" means within 1, 2, 3, or 4 standard deviations. In certain embodiments, the term "about" or "approximately" means within 50%, 20%, 15%, 10%, 9%, 8%, 7%, 6%, 5%, 4%, 3%, 2%, 1%, 0.5%, or 0.05% of a given value or range.

In this specification, unless otherwise indicated, all numerical parameters are to be understood as being prefaced and modified in all instances by the term "about," in which the numerical parameters possess the inherent variability characteristic of the underlying measurement techniques used to determine the numerical value of the parameter. At the very least, and not as an attempt to limit the application of the doctrine of equivalents to the scope of the claims, each numerical parameter described herein should at least be construed in light of the number of reported significant digits and by applying ordinary rounding techniques.

Any numerical range recited herein includes all sub-ranges subsumed within the recited range. For example, a range of "1 to 10" includes all sub-ranges between (and including) the recited minimum value of 1 and the recited maximum value of 10, that is, having a minimum value equal to or greater than 1 and a maximum value equal to or less than 10. Also, all ranges recited herein are inclusive of the end points of the recited ranges. For example, a range of "1 to 10" includes the end points 1 and 10. Any maximum numerical limitation recited in this specification is intended to include all lower numerical limitations subsumed therein, and any minimum numerical limitation recited in this specification is intended to include all higher numerical limitations subsumed therein. Accordingly, Applicant reserves the right to amend this specification, including the claims, to expressly recite any sub-range subsumed within the ranges expressly recited. All such ranges are inherently described in this specification.

In summary, numerous benefits have been described which result from employing the concepts described herein. The foregoing description of the one or more forms has been presented for purposes of illustration and description. It is not intended to be exhaustive or limiting to the precise form disclosed. Modifications or variations are possible in light of the appended claims. The one or more forms were chosen and described in order to illustrate principles and practical application to thereby enable one of ordinary skill in the art to utilize the various forms and with various modifications as are suited to the particular use contemplated. It is intended that the claims submitted herewith define the overall scope.

## Claims

1. A surgical instrument (750), comprising:
an end effector (752, 852), comprising:
a first jaw (753);
a second jaw (753) movable relative to the first jaw between an open configuration and a closed configuration to grasp tissue;
a staple cartridge (767, 867); and
at least one electrode (796, 896);
a drive member (751);
a motor assembly (754) configured to generate drive motions to move the drive member; and
a control circuit (760) configured to:
cause the at least one electrode to deliver (901) a therapeutic energy to the tissue in a first phase of a surgical treatment;
cause the motor assembly to move (903) the drive member to deploy staples (742) from the staple cartridge into the tissue in a second phase of the surgical treatment;
monitor a first tissue property in the first phase of the surgical treatment;
switch from the first phase of the surgical treatment to the second phase of the surgical treatment if at least one of two conditions is met, wherein a first of the two conditions is triggered by reaching or exceeding (902) a predetermined threshold of the first tissue property, and wherein a second of the two conditions is triggered by reaching or exceeding (904) a predetermined threshold time of the first phase;
set (905) a parameter of the second phase of the surgical treatment based on at least one measurement of the tissue property determined in the first phase of the surgical treatment; and
monitor (906) a second tissue property, different from the first tissue property, in the second phase of the surgical treatment.

2. The surgical instrument of Claim 1, wherein the first jaw comprises a channel (744, 844) configured to slidably receive the staple cartridge.

3. The surgical instrument of Claim 2, wherein the channel comprises a cover (740), and wherein the at least one electrode is integral with the cover, optionally wherein the cover comprises a longitudinal opening (741, 841) configured to permit a cartridge deck (730, 830) of the staple cartridge to cooperatively define a tissue contacting surface with the al least one electrode while the staple cartridge is retained in the channel.

4. The surgical instrument of Claim 1, wherein the control circuit is further configured to cause a generator to adjust a level of the therapeutic energy delivered through the at least one electrode based on the first tissue property.

5. The surgical instrument of claim 1, further comprising:
an anvil (766, 866);
a channel (744, 844) configured to receive the staple cartridge; and
an RF overlay (890) pivotally connected to the channel, wherein the RF overlay comprises the at least one electrode.

6. The surgical instrument of Claim 1 or Claim 5, wherein the parameter of the second phase is a drive velocity of the motor assembly.

7. The surgical instrument of Claim 6, wherein the first tissue property is a tissue impedance.

8. The surgical instrument of Claim 7, wherein the at least one measurement is taken at a beginning of the first phase of the surgical treatment or an end of the first phase of the surgical treatment.

9. The surgical instrument of Claim 7, wherein the at least one measurement comprises multiple measurements of the tissue property taken during the first phase of the surgical treatment.

10. The surgical instrument of Claim 5, wherein the anvil is configured to pivot the RF overlay toward a locking engagement with the staple cartridge in the channel.

11. The surgical instrument of Claim 10, wherein the staple cartridge comprises a cartridge deck including a depressed region (831) configured to releasably retain the RF overlay.

12. The surgical instrument of Claim 5, wherein the staple cartridge comprises a latch member (861) configured to lockingly engage a distal portion of the RF overlay.

13. A surgical instrument (750), comprising:
an end effector (752), comprising:
a first jaw (753);
a second jaw (753) movable relative to the first jaw between an open configuration and a closed configuration to grasp tissue;
a staple cartridge (767), comprising:
a longitudinal slot (759); and
staple cavity rows (757a, 757b) extending in consecutive treatment zones alongside the longitudinal slot; and
electrode segments (696a-696f) separately residing in the consecutive treatment zones;
a drive member (751);
a motor assembly (754) configured to generate drive motions to move the drive member; and
a control circuit (760) configured to:
deliver (601) a therapeutic energy to the tissue in all the consecutive treatment zones;
cause (602) the motor assembly to move the drive member to motivate staple deployment from the staple cartridge;
detect (609) a parameter indicative of progress of the staple deployment from the staple cartridge in the consecutive treatment zones; and
sequentially deactivate (610) the electrodes to sequentially seize the delivery of the therapeutic energy to the tissue in the consecutive treatment zones based on the progress of the staple deployment from the staple cartridge.

14. The surgical instrument of Claim 13, wherein the electrode segments comprise:
a proximal electrode segment residing in a proximal treatment zone of the consecutive treatment zones; and
a distal electrode segment residing in a distal treatment zone of the consecutive treatment zones, wherein a detected completion of the staple deployment from the staple cartridge in the proximal treatment zone causes the control circuit to deactivate the proximal electrode segment.

15. The surgical instrument of Claim 14, wherein a detected completion of the staple deployment from the staple cartridge in the distal treatment zone causes the control circuit to deactivate the distal electrode segment.

## Patentansprüche

1. Chirurgisches Instrument (750), umfassend:
einen Endeffektor (752, 852), umfassend:
eine erste Backe (753);
eine zweite Backe (753), die im Verhältnis zu der ersten Backe zwischen einer offenen Konfiguration und einer geschlossenen Konfiguration beweglich ist, um Gewebe zu ergreifen;
ein Klammermagazin (767, 867); und
mindestens eine Elektrode (796, 896);
ein Antriebselement (751);
eine Motoranordnung (754), die konfiguriert ist, um Antriebsbewegungen zum Bewegen des Antriebselements zu erzeugen; und
eine Steuerschaltung (760), die konfiguriert ist zum:
Veranlassen, dass die mindestens eine Elektrode (901) in einer ersten Phase einer chirurgischen Behandlung eine therapeutische Energie an das Gewebe abgibt;
Veranlassen, dass die Motoranordnung das Antriebselement bewegt (903), um in einer zweiten Phase der chirurgischen Behandlung Klammern (742) aus dem Klammermagazin in das Gewebe einzusetzen;
Überwachen einer ersten Gewebeeigenschaft in der ersten Phase der chirurgischen Behandlung;
Umschalten aus der ersten Phase der chirurgischen Behandlung in die zweite Phase der chirurgischen Behandlung, wenn mindestens eine von zwei Bedingungen erfüllt ist, wobei eine erste der zwei Bedingungen durch das Erreichen oder Überschreiten (902) eines vorgegebenen Schwellenwerts der ersten Gewebeeigenschaft ausgelöst wird und wobei eine zweite der zwei Bedingungen durch das Erreichen oder Überschreiten (904) einer vorgegebenen Schwellenwertzeit der ersten Phase ausgelöst wird;
Festlegen (905) eines Parameters der zweiten Phase der chirurgischen Behandlung basierend auf mindestens einer Messung der Gewebeeigenschaft, die in der ersten Phase der chirurgischen Behandlung ermittelt wurde; und
Überwachen (906) einer zweiten Gewebeeigenschaft, die sich von der ersten Gewebeeigenschaft unterscheidet, in der zweiten Phase der chirurgischen Behandlung.

2. Chirurgisches Instrument nach Anspruch 1, wobei die erste Backe einen Kanal (744, 844) umfasst, der konfiguriert ist, um das Klammermagazin verschiebbar aufzunehmen.

3. Chirurgisches Instrument nach Anspruch 2, wobei der Kanal eine Abdeckung (740) umfasst und wobei die mindestens eine Elektrode integral mit der Abdeckung ausgebildet ist, wobei die Abdeckung optional eine Längsöffnung (741, 841) umfasst, die konfiguriert ist, um einem Magazindeckel (730, 830) des Klammermagazins zu ermöglichen, mit der mindestens einen Elektrode eine Gewebekontaktfläche zu definieren, während das Klammermagazin in dem Kanal festgehalten wird.

4. Chirurgisches Instrument nach Anspruch 1, wobei die Steuerschaltung ferner konfiguriert ist, um einen Generator zu veranlassen, das Niveau der über die mindestens eine Elektrode abgegebenen therapeutischen Energie basierend auf der ersten Gewebeeigenschaft anzupassen.

5. Chirurgisches Instrument nach Anspruch 1, ferner umfassend:
einen Amboss (766, 866);
einen Kanal (744, 844), der konfiguriert ist, um das Klammermagazin aufzunehmen; und
eine HF-Auflage (890), die schwenkbar mit dem Kanal verbunden ist, wobei die HF-Auflage die mindestens eine Elektrode umfasst.

6. Chirurgisches Instrument nach Anspruch 1 oder Anspruch 5, wobei der Parameter der zweiten Phase eine Antriebsgeschwindigkeit der Motoranordnung ist.

7. Chirurgisches Instrument nach Anspruch 6, wobei die erste Gewebeeigenschaft eine Gewebeimpedanz ist.

8. Chirurgisches Instrument nach Anspruch 7, wobei die mindestens eine Messung zu Beginn der ersten Phase der chirurgischen Behandlung oder am Ende der ersten Phase der chirurgischen Behandlung vorgenommen wird.

9. Chirurgisches Instrument nach Anspruch 7, wobei die mindestens eine Messung mehrere Messungen der Gewebeeigenschaft umfasst, die während der ersten Phase der chirurgischen Behandlung vorgenommen werden.

10. Chirurgisches Instrument nach Anspruch 5, wobei der Amboss konfiguriert ist, um die HF-Auflage in Richtung eines Verriegelungseingriffs mit dem Klammermagazin in dem Kanal zu schwenken.

11. Chirurgisches Instrument nach Anspruch 10, wobei das Klammermagazin einen Magazindeckel umfasst, das einen vertieften Bereich (831) einschließt, der konfiguriert ist, um die HF-Auflage lösbar festzuhalten.

12. Chirurgisches Instrument nach Anspruch 5, wobei das Klammermagazin ein Riegelelement (861) umfasst, das konfiguriert ist, um verriegelnd in einen distalen Abschnitt der HF-Auflage einzugreifen.

13. Chirurgisches Instrument (750), umfassend:
einen Endeffektor (752), umfassend:
eine erste Backe (753);
eine zweite Backe (753), die im Verhältnis zu der ersten Backe zwischen einer offenen Konfiguration und einer geschlossenen Konfiguration beweglich ist, um Gewebe zu ergreifen;
ein Klammermagazin (767), umfassend:
einen Längsschlitz (759); und
Klammerhohlraumreihen (757a, 757b), die sich in aufeinanderfolgenden Behandlungszonen entlang des Längsschlitzes erstrecken; und
Elektrodensegmente (696a bis 696f), die sich separat in den aufeinanderfolgenden Behandlungszonen befinden;
ein Antriebselement (751);
eine Motoranordnung (754), die konfiguriert ist, um Antriebsbewegungen zum Bewegen des Antriebselements zu erzeugen; und
eine Steuerschaltung (760), die konfiguriert ist zum:
Abgeben (601) einer therapeutischen Energie an das Gewebe in allen aufeinanderfolgenden Behandlungszonen;
Veranlassen (602), dass die Motoranordnung das Antriebselement bewegt, um das Einsetzen von Klammern aus dem Klammermagazin zu motivieren;
Erkennen (609) eines Parameters, der den Fortschritt des Einsetzens von Klammern aus dem Klammermagazin in den aufeinanderfolgenden Behandlungszonen angibt; und
aufeinanderfolgenden Deaktivieren (610) der Elektroden, um die Abgabe der therapeutischen Energie an das Gewebe in den aufeinanderfolgenden Behandlungszonen basierend auf dem Fortschritt der Klammereinsetzung aus dem Klammermagazin nacheinander zu beenden.

14. Chirurgisches Instrument nach Anspruch 13, wobei die Elektrodensegmente umfassen:
ein proximales Elektrodensegment, das sich in einer proximalen Behandlungszone der aufeinanderfolgenden Behandlungszonen befindet, und
ein distales Elektrodensegment, das sich in einer distalen Behandlungszone der aufeinanderfolgenden Behandlungszonen befindet, wobei ein erkannter Abschluss der Klammereinsetzung aus dem Klammermagazin in der proximalen Behandlungszone bewirkt, dass die Steuerschaltung das proximale Elektrodensegment deaktiviert.

15. Chirurgisches Instrument nach Anspruch 14, wobei eine erkannte Beendigung der Klammereinsetzung aus dem Klammermagazin in der distalen Behandlungszone veranlasst, dass die Steuerschaltung das distale Elektrodensegment deaktiviert.

## Revendications

1. Instrument chirurgical (750), comprenant :
un effecteur terminal (752, 852), comprenant :
une première mâchoire (753) ;
une seconde mâchoire (753) mobile par rapport à la première mâchoire entre une configuration ouverte et une configuration fermée pour saisir un tissu ;
une cartouche d'agrafes (767, 867) ; et
au moins une électrode (796, 896) ;
un élément d'entraînement (751) ;
un ensemble moteur (754) conçu pour générer des mouvements d'entraînement afin de déplacer l'élément d'entraînement ; et
un circuit de commande (760) configuré pour :
amener l'au moins une électrode à délivrer (901) une énergie thérapeutique au tissu au cours d'une première phase d'un traitement chirurgical ;
amener l'ensemble moteur à déplacer (903) l'élément d'entraînement pour déployer des agrafes (742) de la cartouche d'agrafes dans le tissu au cours d'une seconde phase du traitement chirurgical ;
surveiller une première propriété tissulaire au cours de la première phase du traitement chirurgical ;
passer de la première phase du traitement chirurgical à la seconde phase du traitement chirurgical si au moins l'une parmi les deux conditions est satisfaite, dans lequel la première des deux conditions est déclenchée par l'atteinte ou le dépassement (902) d'un seuil prédéterminé de la première propriété tissulaire, et la seconde des deux conditions est déclenchée par l'atteinte ou le dépassement (904) d'un seuil temporel prédéterminé de la première phase ;
définir (905) un paramètre de la seconde phase du traitement chirurgical sur la base d'au moins une mesure de la propriété tissulaire déterminée au cours de la première phase du traitement chirurgical ; et
surveiller (906) une seconde propriété tissulaire, différente de la première propriété tissulaire, au cours de la seconde phase du traitement chirurgical.

2. Instrument chirurgical selon la revendication 1, dans lequel la première mâchoire comprend un canal (744, 844) conçu pour recevoir de manière coulissante la cartouche d'agrafes.

3. Instrument chirurgical selon la revendication 2, dans lequel le canal comprend un couvercle (740), et dans lequel l'au moins une électrode fait partie intégrante du couvercle, éventuellement, dans lequel le couvercle comprend une ouverture longitudinale (741, 841) conçue pour permettre à un plateau de cartouche (730, 830) de la cartouche d'agrafes de définir de manière coopérative une surface de contact avec le tissu avec l'au moins électrode pendant que la cartouche d'agrafes est retenue dans le canal.

4. Instrument chirurgical selon la revendication 1, dans lequel le circuit de commande est en outre configuré pour amener un générateur à ajuster un niveau de l'énergie thérapeutique délivrée par le biais d'au moins une électrode sur la base de la première propriété tissulaire.

5. Instrument chirurgical selon la revendication 1, comprenant en outre :
une enclume (766, 866) ;
un canal (744, 844) conçu pour recevoir la cartouche d'agrafes ; et
un recouvrement RF (890) relié de manière pivotante au canal, dans lequel le recouvrement RF comprend l'au moins une électrode.

6. Instrument chirurgical selon la revendication 1 ou la revendication 5, dans lequel le paramètre de la seconde phase est une vitesse d'entraînement de l'ensemble moteur.

7. Instrument chirurgical selon la revendication 6, dans lequel la première propriété tissulaire est une impédance tissulaire.

8. Instrument chirurgical selon la revendication 7, dans lequel l'au moins une mesure est prise au début ou à la fin de la première phase du traitement chirurgical.

9. Instrument chirurgical selon la revendication 7, dans lequel l'au moins une mesure comprend de multiples mesures de la propriété tissulaire prises au cours de la première phase du traitement chirurgical.

10. Instrument chirurgical selon la revendication 5, dans lequel l'enclume est conçue pour faire pivoter le recouvrement RF vers une mise en prise par verrouillage avec la cartouche d'agrafes dans le canal.

11. Instrument chirurgical selon la revendication 10, dans lequel la cartouche d'agrafes comprend un plateau de cartouche comportant une région enfoncée (831) conçue pour retenir de manière amovible le recouvrement RF.

12. Instrument chirurgical selon la revendication 5, dans lequel la cartouche d'agrafes comprend un élément de loquet (861) conçu pour venir en prise de manière verrouillée dans une partie distale du recouvrement RF.

13. Instrument chirurgical (750), comprenant :
un effecteur (752), comprenant :
une première mâchoire (753) ;
une seconde mâchoire (753) mobile par rapport à la première mâchoire entre une configuration ouverte et une configuration fermée pour saisir un tissu ;
une cartouche d'agrafes (767), comprenant :
une fente longitudinale (759) ; et
des rangées de cavités d'agrafes (757a, 757b) s'étendant dans des zones de traitement consécutives le long de la fente longitudinale ; et
des segments d'électrodes (696a à 696f) résidant séparément dans les zones de traitement consécutives ;
un élément d'entraînement (751) ;
un ensemble moteur (754) conçu pour générer des mouvements d'entraînement afin de déplacer l'élément d'entraînement ; et
un circuit de commande (760) configuré pour :
délivrer (601) une énergie thérapeutique au tissu dans toutes les zones de traitement consécutives ;
amener (602) le déplacement de l'élément moteur pour provoquer un déploiement d'agrafes à partir de la cartouche d'agrafes ;
détecter (609) un paramètre indiquant une progression du déploiement d'agrafes à partir de la cartouche d'agrafes dans les zones de traitement consécutives ; et
désactiver de manière séquentielle (610) les électrodes pour interrompre manière séquentielle la délivrance de l'énergie thérapeutique au tissu dans les zones de traitement consécutives sur la base de la progression du déploiement d'agrafes à partir de la cartouche d'agrafes.

14. Instrument chirurgical selon la revendication 13, dans lequel les segments d'électrodes comprennent :
un segment d'électrode proximal résidant dans une zone de traitement proximale des zones de traitement consécutives ; et
un segment d'électrode distal résidant dans une zone de traitement distale des zones de traitement consécutives, dans lequel la détection de l'achèvement du déploiement d'agrafes à partir de la cartouche d'agrafes dans la zone de traitement proximale amène amène le circuit de commande à désactiver le segment d'électrode proximal.

15. Instrument chirurgical selon la revendication 14, dans lequel la détection de l'achèvement du déploiement d'agrafes à partir de la cartouche d'agrafes dans la zone de traitement distale amène le circuit de commande à désactiver le segment d'électrode distal.
